# EUROPEAN PATENT SPECIFICATION

(11) **EP 2 236 616 B1**
(45) Date of publication and mention of the grant of the patent: **18.12.2013**
(21) Application number: 10006771.9
(22) Date of filing: 02.06.2006
(51) Int. Cl.: C12Q 1/10, C12Q 1/06, G01N 33/569, C12Q 1/04, C12Q 1/14

(54) **Peptide nucleic acid probes for analysis of microorganisms**
Peptid-Nukleinsäure-Sonden zur Untersuchung von Mikroorganismen
Sondes d'acides nucléiques peptidiques destinées à l'analyse de micro-organismes

(30) Priority: 02.06.2005 US 687178 P; 01.08.2005 US 704552 P
(43) Date of publication of application: 06.10.2010
(62) Divisional of application: 06772066.4
(73) Proprietor: AdvanDx, Inc., Woburn, MA 01801 (US)
(72) Inventor: Fiandaca, Marc, Princeton, MA 01541 (US); Stender, Henrik, 2600 Glostrup (DK)
(74) Representative: Hiebl, Inge Elisabeth

(56) References cited:
- US-B1- 6 562 958
- DATABASE Geneseq [Online] 21 April 2005 (2005-04-21), XP002596687 retrieved from EBI accession no. GSN:ADW75829 Database accession no. ADW75829
- DATABASE EMBL [Online] 18 June 2002 (2002-06-18), "Sequence 34 from Patent WO0196604." XP002596688 retrieved from EBI accession no. EMBL:AX427070 Database accession no. AX427070
- PERRY-O'KEEFE HEATHER ET AL: "Identification of indicator microorganisms using a standardized PNA FISH method" JOURNAL OF MICROBIOLOGICAL METHODS, ELSEVIER, AMSTERDAM, NL LNKD- DOI:10.1016/S0167-7012(01)00303-7, vol. 47, no. 3, 1 December 2001 (2001-12-01), pages 281-292, XP002228739 ISSN: 0167-7012

### SEQUENCE LISTING

<110> ADVANDX, INC.
<120> PEPTIDE NUCLEIC ACID PROBES FOR ANALYSIS OF MICROORGANISMS
<130> 18294EP1
<140>
   <141> 2006-06-02
<150> 60/687,178 <151> 2005-06-02
<150> 60/704,552 <151> 2005-08-01
<160> 9
<170> PatentIn Ver. 3.3
<210> 1
   <211> 15
   <212> DNA
   <213> Artificial Sequence
<220>
   <223> Description of Artificial Sequence: Synthetic probe
<400> 1
   acacacactg attca 15
<210> 2
   <211> 15
   <212> DNA
   <213> Artificial Sequence
<220>
   <223> Description of Artificial Sequence: Synthetic probe
<400> 2
   cacctacaca ccagc 15
<210> 3
   <211> 15
   <212> DNA
   <213> Artificial Sequence
<220>
   <223> Description of Artificial Sequence: Synthetic probe
<400> 3
   cacttaccat cagcg 15
<210> 4
   <211> 15
   <212> DNA
   <213> Artificial Sequence
<220>
   <223> Description of Artificial Sequence: Synthetic probe
<400> 4
   acaccaaacc tcagc 15
<210> 5
   <211> 15
   <212> DNA
   <213> Artificial Sequence
<220>
   <223> Description of Artificial Sequence: Synthetic probe
<400> 5
   acaccaaaca tcagc 15
<210> 6
   <211> 15
   <212> DNA
   <213> Artificial Sequence
<220>
   <223> Description of Artificial Sequence: Synthetic probe
<400> 6
   ccctagtcgg catag 15
<210> 7
   <211> 15
   <212> DNA
   <213> Artificial Sequence
<220>
   <223> Description of Artificial Sequence: Synthetic probe
<400> 7
   ccaagagatc cgttg 15
<210> 8
   <211> 16
   <212> DNA
   <213> Artificial Sequence
<220>
   <223> Description of Artificial Sequence: Synthetic probe
<400> 8
   cctctcatcg cattac 16
<210> 9
   <211> 14
   <212> DNA
   <213> Artificial Sequence
<220>
   <223> Description of Artificial Sequence: Synthetic probe
<400> 9
   gctcaccagt atcg 14

## Description

### FIELD OF THE INVENTION

The present invention relates to peptide nucleic acid (PNA) probes, PNA probe sets and methods for the analysis of *Acinetobacter* species optionally present in a sample. Microorganisms targeted by the probes of this invention are *Acinetobacter* species. The invention further relates to diagnostic kits comprising such PNA probes.

### BACKGROUND OF THE INVENTION

The diagnosis of infectious diseases is still often based on classical microbiology methodologies, such as culture and biochemical tests for phenotypic markers, and typically takes from 1-2 days up to weeks or even months before final diagnosis is available. In the meantime, patients are often treated empirically based on preliminary test results and clinical symptoms, which often lead to an unnecessary use of antibiotics and its sequelae.

Conventional biochemical methods for the analysis of microorganisms are slow and misidentifications are well known. Rapid and accurate methods for detection, identification and/or quantitation of microorganisms is thus important in order to ensure optimal antibiotic therapy and patient management.

Despite its name, Peptide Nucleic Acid (PNA) is neither a peptide nor a nucleic acid, it is not even an acid. PNA is a non-naturally occuring polyamid that can hybridize to nucleic acid (DNA and RNA) with sequence specificity (see: U.S. Pat. No. 5,539,082) and Egholm et al., Nature 365:566-568 (1993)) according to Watson-Crick base paring rules. However, whereas nucleic acids are biological materials that play a central role in the life of living species as agents of genetic transmission and expression, PNA is a recently developed totally artificial molecule, conceived in the minds of chemists and made using synthetic organic chemistry. PNA also differs structurally from nucleic acid. Although both can employ common nucleobases (A, C, G, T, and U), the backbones of these molecules are structurally diverse. The backbones of RNA and DNA are composed of repeating phosphodiester ribose and 2-deoxyribose units. In contrast, the backbones of the most common PNAs are composed of (aminoethyl)-glycine subunits. Additionally, in PNA the nucleobases are connected to the backbone by an additional methylene carbonyl moiety. PNA is therefore not an acid and therefore contains no charged acidic groups such as those present in DNA and RNA. The non-charged backbone allows PNA probes to hybridize under conditions that are destabilizing to DNA and RNA, attributes that enable PNA probes to access targets, such as highly structured rRNA and double stranded DNA, known to be inaccessible to DNA probes (See: Stephano & Hyldig-Nielsen, IBC Library Series Publication #948. International Business Communication, Southborough, MA, pp. 19-37 (1997)). PNAs are useful candidates for investigation when developing probe-based hybridization assays because they hybridize to nucleic acids with sequence specificity. PNA probes, however are not the equivalent of nucleic acid probes in structure or function.

Comparative analysis of ribosomal RNA (rRNA) sequences or genomic DNA sequences corresponding to said rRNA (rDNA) has become a widely accepted method for establishing phylogenetic relationships between bacterial species (Woese, Microbiol. Rev. 51:221-271 (1987)), and Bergey's Manual of systematic bacteriology has been revised based on rRNA or rDNA sequence comparisons. Ribosomal RNA or rDNA sequence differences between closely related species enable design of specific probes for microbial identification and thus enable diagnostic microbiology to be based on a single genetic marker rather than a series of phenotypic markers as characterizing traditional microbiology (Delong et al., Science 342:1360-1363 (1989)).

PNA fluorescent *in situ* hybridization (PNA FISH) technology for detection of specific microorganisms has been described in several articles (see for example Stender et al, J Clin Microbiol. 1999 Sep;37(9):2760-5; Rigby et al J Clin Microbiol. 2002 Jun;40(6):2182-6; and Oliveira et al J Clin Microbiol. 2002 Jan;40(1):247-51.) and several kits are commercially available (AdvanDx, Inc, Wobum MA). PNA FISH as its name indicates uses fluorescent labeled PNA probes to illuminate samples based on the specific hybridization of PNA probes to nucleic acid targets in the sample. Usually PNA probes are designed to detect high copy number targets such as ribosomal RNA. The PNA FISH method is accepted as an improvement over traditional microbiological detection techniques in that it has the potential to provide rapid and accurate identification of microorganisms to the species level. One limitation of PNA FISH technology is the availability of proven probe sequences. Since there is some expense required to manufacture and test novel probes, and there is some art to the design of probes, novel probe sequences of proven value are infrequently described.

US 6,562,958 discloses isolated polypeptide and nucleic acid sequences derived from *Acinetobacter mirabilis* that are useful in diagnosis and therapy of pathological conditions as well as antibodies against said polypetides and methods for producing said polypeptides.

Further disclosed are methods for the detection, prevention and treatment of pathological conditions resulting from bacterial infection.

Perry-O'Keefe, H. et al.: "Identification of indicator microorganisms using a standardized PNA FISH method" (2001-12-01, vol. 47, no. 3, pp. 281-292) discloses the development of a standardized fluorescent *in situ* hybridization (FISH) method using peptide Nucleic Acid (PNA) probes for analysis of gram-negative and gram-positive bacteria as well as yeasts.

EBI accession number GSN:ADW75829 (2005-04-21) shows a 25 bp sequence from *Arabidopsis thaliana.*

EBI accession number EMBL:AX427070 (2002-06-18) shows a 19 bp artificial sequence corresponding to SEQ ID NO:34 of WO 01/96604.

IBIS analysis (query = EP10006771:8) identifies a 13 bp sequence from *Homo sapiens* with GENESEQ accession number ABF41701, forming part of a sequence disclosed in WO 2001/77384.

IBIS analysis (query = EP10006771:9) identifies a 14 bp sequence from *Homo sapiens* with EMBL accession number AX030102, forming part of a sequence disclosed in EP 1008649.

### SUMMARY OF THE INVENTION

This invention is directed to a PNA probe comprising a nucleobase sequence for the analysis of *Acinetobacter* species, wherein said PNA probe is SEQ ID NO: 8 or 9 or a complement thereof. This invention is further directed to a method for the detection, identification or quantitation of *Acinetobacter* species in a sample, said method comprising contacting at least one PNA probe according to this invention to the sample, hybridizing the PNA probe to a target sequence of *Acinetobacter* species in the sample and detecting the hybridization as being indicative of presence, identity and/or amount of *Acinetobacter* species in the sample. The invention is further directed to a kit for detection, identification or quantitation of one or more *Acinetobacter* species comprising one or more PNA probes according to this invention and instructions for use. In accordance with the invention, the PNA probes are directed to rRNA or the genomic sequences corresponding to said rRNA (rDNA) or its complement. In preferred embodiments the probes of this invention are used for *in situ* hybridization analysis of *Acinetobacter* species optionally present in a sample, most preferably the *in situ* hybridization analysis is fluorescence *in situ* hybridization

In one embodiment, this invention is directed to PNA probes for detection, identification and/or quantitation of

### Acinetobacter species.

These PNA probes have the inherent physicolchemical characteristics of PNA probes as compared to nucleic acid probes, such that rapid and accurate analysis can be performed using just a single PNA probe. Furthermore, many microorganisms have a relatively rigid cell wall where the improved penetration of PNA probes also offers an advantage as compared to nucleic acid probes when applied in fluorescence *in situ* hybridization assays. Where nucleic acid probes require fixation and permeabilization with cross-linking agents and/or enzymes (for example see Kempf et al., J. Clin. Microbiol 38:830-838 (2000)), these PNA probes can be applied directly following smear preparation as exemplified in example 1.

According to the present invention the PNA probe is SEQ ID NO: 8 (16 nucleobases) or SEQ ID NO: 9 (14 nucleobases) Naturally occurring nucleic acid probes are typically at least 18 nucleobases (For example see Kempf et al., J. Clin. Microbiol 38:830-838 (2000)) due to their lower Tm values. This difference provides these PNA probes with better discrimination to closely related non-target sequences with only a single or just a few nucleobase difference(s) as required for analysis of rRNA or rDNA from microorganisms.

PNA probe nucleobase sequences according to the invention are selected from the group consisting of:
CCT-CTC-ATC-GCA-TTA-C (SEQ ID NO:8), and GCT-CAC-CAG-TAT-CG (SEQ ID NO:9). One or more of these probes, or the complements thereof, are included in the probe sets of this invention.

Preferably probes of this invention are labeled with at least one detectable moiety, wherein the detectable moiety or moieties are selected from the group consisting of: a conjugate, a branched detection system, a chromophore, a fluorophore, a spin label, a radioisotope, an enzyme, a hapten, an acridinium ester and a luminescent compound. Fluorescent labeled probes of this invention may be self-reporting, most preferably self-reporting fluorescent probes of this invention are PNA Linear Beacons. Probes of this invention may contain two or more fluorescent labels which are independently detectable, or detectable by coincidental fluorescence.

In other conceptions of the present invention PNA probes are included which contain moieties that add functionality to the probe. Such moieties include but are not limited to spacer and linker g roups. Likewise, PNA probes of this invention encompass probes attached to a solid support such as but not limited to a membrane, a slide, an array, a bead, or a particle.

Probe sets of this invention include two or more PNA probes for the analysis of *Acinetobacter* species optionally present in a sample. Probe sets are preferably labeled with a detectable moiety. Probe sets may be labeled with the same detectable moiety, or they may be differently labeled for independent analysis of probe signals. It is within the conception of this invention that two or more differently labeled fluorescent probes of a probe set may be used to create a third signal by coincidental fluorescence.

The method according to the invention comprises contacting a sample with one or more of the PNA probes described above. According to the method, the presence, absence and/or number of *Acinetobacter* species in the sample is then detected, identified and/or quantitated.

Consequently, the analysis is based on a single assay with a definitive outcome. In contrast, current routine methods for analysis of microorganisms are based on multiple phenotypic characteristics involving multiple tests.

In preferred embodiments the methods of this invention are used for *in situ* hybridization analysis of microorganisms optionally present in a sample, most preferably the *in situ* hybridization analysis is fluorescence *in situ* hybridization analysis. In preferred methods of the invention, the sample is a biological sample, including but not limited to blood, urine, secretion, sweat, sputum, stool, mucous, or cultures thereof.

Preferred methods of the invention optionally include non-labeled blocking probes to reduce or eliminate hybridization of PNA probes to non-target sequences. Methods of this invention do not include the use of cross-linking reagents or enzymes prior to hybridization.

The methods of this invention may also be used to detect nucleic acid targets generated, synthesized or amplified in a reaction. Preferred methods for generating, synthesizing or amplifying targets include PCR, LCR, SDA, TMA, RCA and Q-beta replicase.

Methods of the invention include those in which the targets are immobilized to a surface, such as a membrane, a slide, a bead, or a particle and which may furthermore be a component of an array. Optionally, the methods may include PNA probes which are immobilized to a surface such as a membrane, a slide, a bead, or a particle, and may furthermore be a component of an array.

In still another embodiment, this invention is directed to kits suitable for performing an assay that detect, identify and/or quantitate *Acinetobacter* species optionally present in a sample.

The kits of this invention comprise one or more PNA probes and other reagents or compositions that are selected to perform an assay or otherwise simplify the performance of an assay. Preferred kit formats include kits designed to perform *in situ* hybridization assays, and kits designed to perform real-time PCR assays. Preferred kits are designed to examine samples such as clinical specimens, or cultures thereof.

The PNA probes, methods and kits of this invention have been demonstrated to be both sensitive and specific for *Acinetobacter* species they are directed to. Moreover, the assays described herein are rapid (less than 3 hours) and capable of analysis of *Acinetobacter* species in a single assay.

Those of ordinary skill in the art will also appreciate that the complement probing sequence is equally suitable for assays, such as but not limited to real-time PCR, that are using rDNA as target.

### DETAILED DESCRIPTION OF THE INVENTION

### I. Definitions:

a. As used herein, the term "nucleobase" means those naturally occurring and those non-naturally occurring heterocyclic moieties commonly known to those who utilize nucleic acid technology or utilize peptide nucleic acid technology to thereby generate polymers that can sequence specifically bind to nucleic acids.
   Non-limiting examples of suitable nucleobases include, but are not limited to: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-azaguanine) and N8-(7-deaza-8-aza-adenine). Other non-limiting examples of suitable nucleobase include those nucleobases illustrated in FIGS. 2(A) and 2(B) of Buchardt et al. (U.S. Pat. No. 6,357,163 or WO92/20702 or WO92/20703) ).
b. As used herein, the term "nucleobase sequence" means any segment of a polymer that comprises nucleobase-containing subunits. Non-limiting examples of suitable polymers or polymer segments include oligodeoxynucleotides, oligoribonucleotides, peptide nucleic acids, nucleic acid analogs, nucleic acid mimics, and/or chimeras.
c. As used herein, the term "target sequence" means the nucleobase sequence that is to be detected in an assay.
d. As used herein, the term "probe" means a polymer (e. g. a DNA, RNA, PNA, chimera or linked polymer) having a probing nucleobase sequence that
   is designed to sequence-specifically hybridize to a target sequence of a target molecule of an organism of interest.
e. As used herein, "analyze" means that the individual bacteria are marked for detection, identification and/or quantitation
f. As used herein, "peptide nucleic acid" or "PNA" means any oligomer or polymer segment comprising two or more PNA subunits (residues), including, but not limited to, any of the oligomer or polymer segments referred to or claimed as a peptide nucleic acid in any one or more of U.S. Pat. Nos. 5,539,082, 5,527,675, 5,623,049, 5,714,331, 5,718,262, 5,736,336, 5,773,571, 5,766,855, 5,786,461, 5,837,459, 5,891,625, 5,972,610, 5,986,053, 6,107,470, 6,201,103, 6,350, 853, 6,357,163, 6,395,474, 6,414,112, 6,441,130, 6,451,968; 6969766, and 7022851
   . The term "peptide nucleic acid" or "PNA" shall also apply to any oligomer or polymer segment comprising two or more subunits of those nucleic acid mimics described in the following publications: Lagriffoui et al., Bioorganic & Medicinal Chemistry Letters, 4: 1081-1082 (1994); Petersen et al., Bioorganic & Medicinal Chemistry Letters, 6: 793-796 (1996); Diderichsen et al., Tett. Lett. 37: 475-478 (1996); Fujii et al., Bioorg. Med. Chem. Lett. 7: 637-627 (1997); Jordan et al Bioorg. Med. Chem. Lett. 7: 687-690 (1997); Krotz et al., Tett. Lett. 36: 6941-6944 (1995); Lagriffoul et al., Bioorg. Med. Chem. Lett. 4: 1081-1082 (1994); Diederichsen, U., Bioorganic & Medicinal Chemistry Letters, 7: 1743-1746 (1997); Lowe et al., J. Chem. Soc. Perkin Trans. 1, (1997) 1: 539-546; Lowe et al., J. Chem. Soc. Perkin Trans. 11: 547-554 (1997); Lowe et al., J. Chem. Soc. Perkin Trans. 1 1:5 55-560 (1997); Howarth et al., J. Org. Chem. 62: 5441-5450 (1997); Altmann, K-H et al., Bioorganic & Medicinal Chemistry Letters, 7: 1119-1122 (1997); Diederichsen, U., Bioorganic & Med. Chem. Lett., 8: 165-168 (1998); Diederichsen et al., Angew. Chem. Int. Ed., 37: 302-305 (1998); Cantin et al., Tett. Lett., 38: 4211-4214 (1997); Ciapetti et al., Tetrahedron, 53: 1167-1176 (1997); Lagriffoule et al., Chem. Eur. J., 3: 912-919 (1997); Kumar et al., Organic Letters 3(9): 1269-1272 (2001); and the Peptide-Based Nucleic Acid Mimics (PENAMs) of Shah et al. as disclosed in WO96/04000.. In the most preferred embodiment, a PNA subunit consists of a naturally occurring or non-naturally occurring nucleobase attached to the aza nitrogen of the N- [2-(aminoethyl)] glycine backbone through a methylene carbonyl linkage.
g. As used herein, the terms "label" and "detectable moiety" are interchangeable and shall refer to moieties that can be attached to a probe to thereby render the probe detectable by an instrument or method.
h. As used herein, the term "coincidental fluorescence" is used to describe the perception of a color which is generated by the simultaneous detection of light emissions of two or more labels located near enough in space so as to be irresolvable. The detection of coincidental fluorescence can be either by eye or a photon-sensitive device.

### 2. Description

### I. General:

### PNA Synthesis:

Methods for the chemical assembly of PNAs are well known (See: U.S. Pat. Nos. 5,539,082, 5,527,675, 5,623,049, 5,714,331, 5,718,262, 5,736,336, 5,773,571, 5,766,855, 5,786,461, 5,837,459, 5,891,625, 5,972,610, 5,986,053, 6,107,470, 6,201,103, 6,350, 853, 6,357,163, 6,395,474, 6,414,112, 6,441,130, 6,451,968, 6,969,766, and 7,022,851; (Also see: PerSeptive Biosystems and/or Applied Biosystems Product Literature)). As a general reference for PNA synthesis methodology also please see: Nielsen et al., Peptide Nucleic Acids; Protocols and Applications, Horizon Scientific Press, Norfolk England (1999).

Chemicals and instrumentation for the support bound automated chemical assembly of peptide nucleic acids are now commercially available. Both labeled and unlabeled PNA oligomers are likewise available from commercial vendors of custom PNA oligomers. Chemical assembly of a PNA is analogous to solid phase peptide synthesis, wherein at each cycle of assembly the oligomer possesses a reactive alkyl amino terminus that is condensed with the next synthon to be added to the growing polymer.

PNA may be synthesized at any scale, from submicromole to millimole, or more. PNA can be conveniently synthesized at the 2 µmole scale, using Fmoc(Bhoc) protecting group monomers on an Expedite Synthesizer (Applied Biosystems) using a XAL, PAL or many other suitable commercially available peptide synthesis supports. Alternatively, the Model 433A Synthesizer (Applied Biosystems) with a suitable solid support (e.g. MBHA support) can be used. Moreover, many other automated synthesizers and synthesis supports can be utilized. Synthesis can be performed using continuous flow method and/or a batch method. PNA can also be manually synthesized.

### PNA Labeling:

Preferred non-limiting methods for labeling PNAs are described in US 6,110,676, 6,361,942, 6, 355,421, 6,969,766, and 7,022,851 the examples section of this specification or are otherwise well known in the art of PNA synthesis and peptide synthesis.

### Labels:

Non-limiting examples of detectable moieties (labels) suitable for labeling PNA probes used in the practice of this invention would include a dextran conjugate, a branched nucleic acid detection system, a chromophore, a fluorophore, a spin label, a radioisotope, an enzyme, a hapten, an acridinium ester and a chemiluminescent compound.

Other suitable labeling reagents and preferred methods of attachment would be recognized by those of ordinary skill in the art of PNA, peptide or nucleic acid synthesis.

Preferred haptens include 5 (6)-carboxyfluorescein, 2,4-dinitrophenyl, digoxigenin, and biotin.

Preferred fluorochromes (fluorophores) include 5 (6)-carboxyfluorescein (Flu), 6-((7- amino-4-methylcoumarin-3-acetyl) amino) hexanoic acid (Cou), 5 (and 6)-carboxy-X- rhodamine (Rox), Cyanine 2 (Cy2) Dye, Cyanine 3 (Cy3) Dye, Cyanine 3.5 (Cy3.5) Dye, Cyanine 5 (Cy5) Dye, Cyanine 5.5 (Cy5.5) Dye Cyanine 7 (Cy7) Dye, Cyanine 9 (Cy9) Dye (Cyanine dyes 2,3,3.5,5 and 5.5 are available as NHS esters from Amersham, Arlington Heights, IL), JOE, Tamara or the Alexa dye series (Molecular Probes, Eugene, OR).

Preferred enzymes include polymerases (e. g. Taq polymerase, Klenow PNA polymerase, T7 DNA polymerase, Sequenase, DNA polymerase 1 and phi29 polymerase), alkaline phosphatase (AP), horseradish peroxidase (HRP) and most preferably, soy bean peroxidase (SBP).

### Unlabeled Probes:

The probes that are used for the practice of this invention need not be labeled with a detectable moiety to be operable within the methods of this invention, for example when attached to a solid support

### Self-Indicating Probes:

Beacon probes are examples of self-indicating probes which include a donor moiety and a acceptor moiety. The donor and acceptor moieties operate such that the acceptor moieties accept energy transferred from the donor moieties or otherwise quench signal from the donor moiety. Though the previously listed fluorophores (with suitable spectral properties) might also operate as energy transfer acceptors, preferably, the acceptor moiety is a quencher moiety. Preferably, the quencher moiety is a non-fluorescent aromatic or heteroaromatic moiety. The preferred quencher moiety is 4-((-4-(dimethylamino) phenyl) azo) benzoic acid (dabcyl). In a preferred embodiment, the self-indicating Beacon probe is a PNA Linear Beacon as more fully described in US 6,485, 901.

In another embodiment, the self-indicating probes of this invention are of the type described in WIPO patent application WO97/45539. These self-indicating probes differ as compared with Beacon probes primarily in that the reporter must interact with the nucleic acid to produce signal.

### Spacer/Linker Moieties:

Generally, spacers are used to minimize the adverse effects that bulky labeling reagents might have on hybridization properties of probes. Preferred spacer/linker moieties for the nucleobase polymers of this invention consist of one or more aminoalkyl carboxylic acids (e. g. aminocaproic acid), the side chain of an amino acid (e. g. the side chain of lysine or omithine), natural amino acids (e. g. glycine), aminooxyalkylacids (e. g. 8-amino-3,6-dioxaoctanoic acid), alkyl diacids (e. g. succinic acid), alkyloxy diacids (e. g. diglycolic acid) or alkyldiamines (e. g. 1, 8-diamino-3, 6-dioxaoctane).

### Hybridization Conditions/Stringency:

Those of ordinary skill in the art of nucleic acid hybridization will recognize that factors commonly used to impose or control stringency of hybridization include formamide concentration (or other chemical denaturant reagent), salt concentration (i.e., ionic strength), hybridization temperature, detergent concentration, pH and the presence or absence of chaotropes. Optimal stringency for a probe/target sequence combination is often found by the well known technique of fixing several of the aforementioned stringency factors and then determining the effect of varying a single stringency factor. The same stringency factors can be modulated to thereby control the stringency of hybridization of a PNA to a nucleic acid, except that the hybridization of a PNA is fairly independent of ionic strength. Optimal stringency for an assay may be experimentally determined by examination of each stringency factor until the desired degree of discrimination is achieved.

### Suitable Hybridization Conditions:

Generally, the more closely related the background causing nucleic acid sequences are to the target sequence, the more carefully stringency must be controlled. Blocking probes may also be used as a means to improve discrimination beyond the limits possible by optimization of stringency factors. Suitable hybridization conditions will thus comprise conditions under which the desired degree of discrimination is achieved such that an assay generates an accurate (within the tolerance desired for the assay) and reproducible result. The use of PNA probes presents a notable advantage of the invention over conventional methods using DNA probes. Part of the advantage of PNA is that it hybridizes under conditions which are non-preferred or destabilizing to DNA probes. Examples of such conditions include conditions of low ionic strength (low salt), and high concentrations of lower alcohols, i.e, 50% ethanol.

Aided by no more than routine experimentation and the disclosure provided herein, those of skill in the art will easily be able to determine suitable hybridization conditions for performing assays utilizing the methods and compositions described herein. Suitable *in situ* hybridization or PCR conditions comprise conditions suitable for performing an *in situ* hybridization or PCR procedure. Thus, suitable *in situ* hybridization or PCR conditions will become apparent to those of skill in the art using the disclosure provided herein, with or without additional routine experimentation.

### Blocking Probes:

Blocking probes are nucleic acid or non-nucleic acid probes that can be used to suppress the binding of the probing nucleobase sequence of the probing polymer to a non-target sequence. Preferred blocking probes are PNA probes (see: US 6,110, 676). It is believed that blocking probes operate by hybridization to the non-target sequence to thereby form a more thermodynamically stable complex than is formed by hybridization between the probing nucleobase sequence and the non-target sequence. Formation of the more stable and preferred complex blocks formation of the less stable non-preferred complex between the probing nucleobase sequence and the non-target sequence. Thus, blocking probes can be used with the methods, kits and compositions of this invention to suppress the binding of the probes to a non-target sequence that might be present and interfere with the performance of the assay. Blocking probes are particularly advantageous for. discrimination to the phylogenetically closest related species.

### Probing Nucleobase Sequence:

The probing nucleobase sequence of a probe of this invention is the specific sequence recognition portion of the construct. Therefore, the probing nucleobase sequence is a nucleobase sequence designed to hybridize to a specific target sequence wherein the presence, absence or amount of the target sequence can be used to directly or indirectly detect the presence, absence or number of organisms of interest in a sample. Consequently, with due consideration to the requirements of a probe for the assay format chosen, the length and sequence composition of the probing nucleobase sequence of the probe will generally be chosen such that a stable complex is formed with the target sequence under suitable hybridization conditions.

The probing nucleobase sequences of the probes of this invention that are suitable for the detection, identification and/or enumeration of *Acinetobacter* species comprise nucleobase sequences of: CCT-CTC-ATC-GCA-TTA-C (SEQ ID NO:8), and GCT-CAC-CAG-TAT-CG (SEQ ID NO:9), and the complements thereto.

Complements of the probing nucleobase sequence are considered to be an embodiment of this invention, since it is possible to generate a suitable probe if the target sequence to be detected has been amplified or copied to thereby generate the complement to the identified target sequence.

### Detection, identification and/or enumeration:

By detection is meant analysis for the presence or absence of the organism (= *Acinetobacter* species) optionally present in the sample. By identification is meant establishment of the identity of the organism by genus and species name. By quantitation is meant enumeration of the organisms in a sample. Some assay formats provide simultaneous detection, identification and enumeration (for example see Stender, H. et al., J. Microbiol. Methods. 45:31-39 (2001), others provide detection and identification (for example see Stender, H. et al., Int. J. Tuberc. Lung Dis. 3:830-837 (1999) and yet other assay formats just provide identification (for example see Oliveira, K et al. J. Clin. Microbiol. 40:247-251 (2002)).

### II. Embodiments of the Invention:

### a. PNA Probes:

The PNA probes of this invention are suitable for detecting, identifying and/or quantitating *Acinetobacter* species. General characteristics (e.g. length, labels, nucleobase sequences, linkers etc.) of PNA probes suitable for the analysis have been previously described herein. The probing nucleobase sequence of PNA probes of this invention are listed in Table 1.

**Table 1**

| Sequence ID | Nucleobase sequence |
|---|---|
| SEQ ID NO:8 | CCT-CTC-ATC-GCA-TTA-C |
| SEQ ID NO:9 | GCT-CAC-CAG-TAT-CG |

The PNA probes of this invention may comprise only a probing nucleobase sequence (as previously described herein) or may comprise additional moieties. Non-limiting examples of additional moieties include detectable moieties (labels), linkers, spacers, natural or non-natural amino acids, or other subunits of PNA, DNA or RNA. Additional moieties may be functional or non-functional in an assay. Generally however, additional moieties will be selected to be functional within the design of the assay in which the PNA probe is to be used. The preferred PNA probes of this invention are labeled with one or more detectable moieties selected from the group consisting of fluorophores, enzymes and haptens.

In preferred embodiments, the probes of this invention are used in *in situ* hybridization (ISH) and fluorescence *in situ* hybridization (FISH) assays. Excess probe used in an ISH or FISH assay typically must be removed so that the detectable moiety of the specifically bound probe can be detected above the background signal that results from still present but unhybridized probe. Generally, the excess probe is washed away after the sample has been incubated with probe for a period of time. However, the use of self-indicating probes is a preferred embodiment of this invention, since there is no requirement that excess self-indicating probe be completely removed (washed away) from the sample since it generates little or no detectable background. In addition to ISH or FISH assays, self-indicating probes comprising the selected probing nucleobase sequence described herein are particularly useful in all kinds of homogeneous assays such as in real-time PCR or useful with self-indicating devices (e. g. lateral flow assay) or self-indicating arrays.

### b. PNA probe sets

Probe sets of this invention comprise two of more PNA probes. In one embodiment, some of the PNA probes of the set can be blocking probes. Probes sets may include any group of two or more of the probes of this invention, whether labeled or non-labeled, and may also include probes not specifically described here, but which include at least one of the probes of this invention. The probe set according to the invention is CCT-CTC-ATC-GCA-TTA-C (SEQ ID NO:8), and GCT-CAC-CAG-TAT-CG (SEQ ID NO:9) for detection of *Acinetobacter* species.

### c. Methods:

In another embodiment, this invention is directed to a method suitable for analysis of *Acinetobacter* species optionally in a sample. The general and specific characteristics of PNA probes suitable for the analysis of microorganisms have been previously described herein. The probing nucleobase sequences according to the invention are listed in Table 1.

The method for analysis of *Acinetobacter* species in a sample comprises contacting the sample with one or more PNA probes suitable for hybridization to a target sequence which is specific.

According to the method, the *Acinetobacter* species in the sample is then detected, identified and/or quantitated. This is made possible by correlating hybridization, under suitable hybridization conditions, of the probing nucleobase sequence of a PNA probe to the target sequence of *Acinetobacter* species sought to be detected with the presence, absence or number of the *Acinetobacter* species in the sample. Typically, this correlation is made possible by direct or indirect detection of the probe/target sequence hybrid.

### Fluorescence in situ Hybridization and Real-time PCR:

The PNA probes, methods, kits and compositions of this invention are particularly useful for the rapid probe-based analysis of microorganisms. In preferred embodiments, *in situ* hybridization or PCR is used as the assay format for analysis of microorganisms. Most preferably, fluorescence *in situ* hybridization (PNA FISH) or real-time PCR is the assay format. (Reviewed by Stender et al. J. Microbiol. Methods 48:1-17 (2002)). Preferably, smears for PNA FISH analysis are not treated with cross-linking agents or enzymes prior to hybridization.

### Exemplary Assay Formats:

Exemplary methods for performing PNA FISH can be found in: Oliveira et., J. Clin. Microbiol 40:247-251 (2002), Rigby et al., J. Clin. Microbiol. 40:2182-2186 (2002), Stender et al., J. Clin. Microbiol. 37:2760-2765 (1999), Perry-O'Keefe et al., J. Microbiol. Methods 47:281-292 (2001). According to one method, a smear of the sample, such as, but not limited to, a positive blood culture, is prepared on microscope slides and covered with one drop of the fluorescein-labeled PNA probe in hybridization buffer. A coverslip is placed on the smear to ensure an even coverage, and the slide is subsequently placed on a slide warmer or incubator at 55 °C for 90 minutes. Following hybridization, the coverslip is removed by submerging the slide into a pre-warmed stringent wash solution and the slide is washed for 30 minutes. The smear is finally mounted with one drop of mounting fluid, covered with a coverslip and examined by fluorescence microscopy.

*Acinetobacter* species optimally present in a sample which may be analyzed with the PNA probes contained in the kits of this invention can be determined by several instruments, such as but not limited to the following examples: microscope (for example see Oliveira et al., J. Clin. Microbiol 40:247-251 (2002)), film (for example see Perry-O'Keefe et al., J. Appl. Microbiol. 90:180-189) (2001), camera and instant film (for example see Stender et al., J. Microbiol. Methods 42:245-253 (2000)), luminometer (for example see Stender et al., J. Microbiol. Methods. 46:69-75 (2001), laser scanning device (for example see Stender et al J. Microbiol. Methods. 45: 31-39 (2001) or flow cytometer (for example see Wordon et al., Appl. Environ. Microbiol. 66:284-289 (2000)). Automated slide scanners and flow cytometers are particularly useful for rapidly quantitating the number of microorganisms present in a sample of interest.

Exemplary methods for performing real-time PCR using self-reporting PNA probes can be found in: Fiandaca et al., Abstract, Nucleic Acid-Based technologies. DNA/RNA/PNA Diagnostics, Washington, DC, May 14-16, 2001, and Perry-O'Keefe et al., Abstract, International Conference on Emerging Infectious Diseases, Atlanta, 2002.

### d. Kits:

In yet another embodiment, this invention is directed to kits suitable for performing an assay, which analyses *Acinetobacter* species optionally present in a sample. The general and preferred characteristics of PNA probes suitable for the analysis of *Acinetobacter* species have been previously described herein. Probing nucleobase sequences are listed in Table 1. Furthermore, methods suitable for using the PNA probes to analyze *Acinetobacter* species in a sample have been previously described herein.

The kits of this invention comprise one or more PNA probes and other reagents or compositions which are selected to perform an assay or otherwise simplify the performance of an assay used to analyze microorganisms in a sample.

The kits can, for example, be used for *in situ* assays or for use with nucleic acid amplification technologies. Non-limiting examples of nucleic acid amplification technologies include, but are not limited to, Polymerase Chain Reaction (PCR), Ligase Chain Reaction (LCR), Strand Displacement Amplification (SDA), Transcription-Mediated Amplification (TMA), Q-beta replicase amplification (Q-beta) and Rolling Circle Amplification (RCA).

Accordingly, in some embodiments the other reagents can comprise, buffers, enzymes and/or master mixes for performing an *in situ* or nucleic acid amplification based assay.

### e. Exemplary Applications For Using The Invention:

The PNA probes, methods and kits of this invention are particularly useful for the analysis of *Acinetobacter* species in clinical samples, e.g. urine, blood, wounds, sputum, laryngeal swabs, gastric lavage, bronchial washings, biopsies, aspirates, expectorates as well as in food, beverages, water, pharmaceutical products, personal care products, dairy products or environmental samples and cultures thereof.

### Additional Detection Strategies

Though probes labeled with fluorescein and tamra are described, it is within the concept of this invention that any combination of fluorescent labels could be used which produce a perceivable third color. Likewise, use of two or more labels to produce multiple perceivable colors is also envisioned. Potential fluorescent labels are included in the description. Coincidental fluorescence of two or more fluorescent moieties has been demonstrated to be useful in the generation of a spectrum of colors (Kool et al JACS 2003). Combination colors are made through "mixtures" of two or more fluorophores, and adjustment of their ratios. For example, a combination of two parts red and one part green produces a different color that one part red and two parts green. Though accurate discrimination of these various shades by eye may have a practical limit, it is not difficult to conceive of a device which could accurately perceive such subtle color variations.

### Detectable and Independently Detectable Moieties/Multiplex Analysis:

A multiplex hybridization assay can be performed in accordance with this invention. In a multiplex assay, numerous conditions of interest can be simultaneously examined.

Multiplex analysis relies on the ability to sort sample components or the data associated therewith, during or after the assay is completed. In preferred embodiments of the invention, one or more distinct independently detectable moieties can be used to label two or more different probes used in an assay. The ability to differentiate between and/or quantitate each of the independently detectable moieties provides the means to multiplex a hybridization assay. Correlation of the hybridization of each of the distinctly (independently) labeled probes to particular nucleic acid sequences is indicative of presence, absence or quantity of each organism sought to be detected in the sample.

Consequently, the multiplex assays of this invention can be used to simultaneously detect the presence, absence or quantity of two or more different organisms (i.e. species of *Acinetobacter*) in the same sample and in the same assay. For example, a multiplex assay may utilize two or more PNA probes, each being labeled with an independently detectable fluorophore, or a set of independently detectable fluorophores.

### EXAMPLES

This invention is now illustrated by the following example, which are not intended to be limiting in any way.

### EXAMPLE 1. (referential)

### PNA probe sequence

Kpn23S01-Flu Flu-OO-CACCTACACACCAGC-NH₂ (SEQ ID NO:2)
Kox23S01-Tam Tam-OO-CACTTACCATCAGCG-NH₂ (SEQ ID NO:3)
(Note: Conventional nomenclature used to illustrate the termini of the PNA probe; O = 8-amino-3,6-dioxaoctanoic acids; flu = 5(6)-carboxy-fluorescein, tam = 5(6)-carboxytetramethyrhodamine)

### Bacterium strains

Overnight cultures of reference strains were prepared (American Type Culture Collection, Manassas, VA) representing various Gram negative rods, including *Klebsiella pneumoniae,* and *Klebsiella oxytoca* species.

### Preparation of smears.

For each strain, smears were prepared on a 8-mm diameter well of a teflon-coated microscope slide (AdvanDx, Wobum, MA) by mixing one drop of culture with one drop of phosphate-buffered saline containing 1% (v/v) Triton X-100 . The slide was then placed on a 55°C slide warmer for 20 min at which point the smears were dry. Subsequently, the smears were disinfected by immersion into 96% (v/v) ethanol for 5-10 minutes and air-dried.

### Fluorescence in situ hybridization (FISH).

Smears were covered with a drop of hybridization solution containing 10% (w/v) dextran sulfate, 10 mM NaCl, 30% (v/v) formamide, 0.1% (w/v) sodium pyrophosphate, 0.2% (w/v) polyvinylpyrrolidone, 0.2% (w/v) ficoll, 5 mM Na₂EDTA, 1% (v/v) Triton X-100, 50 mM Tris/HCl pH 7.5 and 500 nM Kpn23S01-Flu and 500 nM Kox23S01-Tam probes (Flu is a fluorescent dye which produces a characteristic green fluorescence, Tam is a fluorescent dye which produces a characteristic red fluorescence). Coverslips were placed on the smears to ensure even coverage with hybridization solution, and the slides were subsequently placed on a slide warmer (Slidemoat, Boekel, Germany) and incubated for 90 min at 55 °C. Following hybridization, the coverslips were removed by submerging the slides into approximately 20 ml/slide pre-warmed 25 mM Tris, pH 10, 137 mM NaCl, 3 mM KCI in a water bath at 55 °C and washed for 30 min. Each smear was finally mounted using one drop of Mounting medium (AdvanDx, Woburn, MA) and covered with a coverslip. Microscopic examination was conducted using a fluorescence microscope equipped with a FITC/Texas Red dual band filter set. *Klebsiella pneumoniae*, was identified by green fluorescent rods and *Klebsiella oxytoca* was identified by red fluorescent rods. Results are recorded in Table 2.

**Table 2**

| Organism | Result |
|---|---|
| *Escherichia coli* | Negative |
| *Klebsiella pneumoniae* | Positive/Green |
| *Klebsiella oxytoca* | Positive/Red |
| *Pseudomonas aeruginosa* | Negative |
| *Pseudomonas florescens* | Negative |
| *Pseudomonas mendocina* | Negative |
| *Pseudomonas mucidolens* | Negative |
| *Pseudomonas pseudoalcigenes* | Negative |
| *Pseudomonas putida* | Negative |
| *Pseudomonas syringe* | Negative |

With reference to Table 2, only two of the ten Gram-negative rod species tested gave a positive result, the *Klebsiella pneumoniae* sample contained green fluorescent rods, and the *Klebsiella oxytoca* sample contained red fluorescent rods. The positive results demonstrate that the probe mixture tested produces species specific signals of green and red with *Klebsiella pneumoniae* and *Klebsiella oxytoca* respectively.

### EXAMPLE 2. (referential)

### PNA probe sequence

Saga16S03-Flu Flu-OO-ACACCAAACCTCAGC (Seq. Id. No.4)
Saga16S04-Flu Flu-OO-ACACCAAACATCAGC (Seq. Id. No.5)
(Note: Conventional nomenclature used to illustrate the termini of the PNA probe; O = 8-amino-3,6-dioxaoctanoic acids; flu = 5(6)-carboxy-fluorescein)

### Bacterium strains

Overnight cultures of reference strains were prepared (American Type Culture Collection, Manassas, VA) representing various Gram positive cocci, including *Streptococcus agalactiae.*

### Preparation of smears.

Smears were prepared as described in Example 1.

### Fluorescence in situ hybridization (FISH).

Hybridization was performed as described in Example 1 except the probes used were either Saga16S03-Flu at 500 nM, Saga16S04-Flu at 500 nM, or a combination of Saga16S03-Flu and Saga16S04-Flu, both at 500 nM ("dual probe"). Microscopic examination was conducted using a fluorescence microscope equipped with a FITC/Texas Red dual band filter set. *Streptococcus agalactiae* was identified by green fluorescent cocci. Results are recorded in Table 3.

**Table 3**

| **Organism** | **Saga16S03** | **Saga16S04** | **Dual Probe** |
|---|---|---|---|
| *Streptococcus agalactiae* | Positive/Green | Negative | Positive/Green |
| *Streptococcus equisimilis* | Negative | Negative | Negative |
| *Streptococcus pyogenes* | Negative | Negative | Negative |
| *Enterococcus durans* | Negative | Negative | Negative |
| *Enterococcus faecalis* | Negative | Negative | Negative |
| *Enterococcus facium* | Negative | Negative | Negative |
| *Enterococcus hirae* | Negative | Negative | Negative |

With reference to Table 3, positive results occurred when either the Saga16S03 probe, or the dual probe set were used to detect *S*. *agalactiae*, all other results were negative indicating that the probes/probe sets are specific.

### EXAMPLE 3. (referential)

### PNA probe sequence

PF-Adx1 Flu-OO-CCCTAGTCGGCATAG (Seq. Id. No.6)
PF-Adx2 Flu-OO-CCAAGAGATCCGTTG (Seq. Id. No.7)
(Note: Conventional nomenclature used to illustrate the termini of the PNA probe; O = 8-amino-3,6-dioxaoctanoic acids; flu = 5(6)-carboxy-fluorescein)

### Bacteria/fungal strains

Overnight cultures of reference strains were prepared (American Type Culture Collection, Manassas, VA) representing various Gram positive cocci, including *Streptococcus agalactiae.*

### Preparation of smears.

Smears were prepared as described in Example 1.

### Fluorescence in situ hybridization (FISH).

Hybridization was performed as described in Example 1 except the probes used were either PF-Adx1 at 500 nM, or PF-Adx2 at 500 nM. Microscopic examination was conducted using a fluorescence microscope equipped with a FITC/Texas Red dual band filter set. Fungi was identified by green fluorescent buds or hyphae. Results are recorded in Table 4.

**Table 4**

| **Organism** | **PF-Adx1** | **PF-Adx2** |
|---|---|---|
| *Candida albicans* | Positive/Green | Positive/Green |
| *Candida catenulata* | Positive/Green | Positive/Green |
| *Candida glabrata* | Positive/Green | Negative |
| *S. aureus* | Negative | Negative |
| *E. coli* | Negative | Negative |
| *Saccharomyces cerevisiae* | Positive/Green | Positive/Green |

With reference to Table 4, all of the fungal organisms tested with the PF-Adx1 probe including *C. albicans*, *C*. *catenulata, C. glabrata,* and *S*. *cerevisiae* were positive. Both of the bacteria species tested (*S. aureus* and *E. coli*) were negative. The PF-Adx2 probe produced a similar pattern of results to PF-Adx1, with the exception of *C*. *glabrata,* which tested negative. Sequence alignment of published *C. glabrata* 5.8S rRNA genes demonstrated that there is a single base mismatch in the gene which is the probable cause of the negative result.

### EXAMPLE 4.

### PNA probe sequence

Eco23S277-Flu Flu-OO-ACA-CAGACT-GAT-TCA (Seq. Id. No.1)
Abau23S-03b-Flu Flu-OO-CCT-CTC-ATC-GCA-TTA-C (Seq. Id. No.8)
Abau16S-04c-Flu Flu-OO-GCT-CAC-CAG-TAT-CG (Seq. Id. No.9)
(Note: Conventional nomenclature used to illustrate the termini of the PNA probe; O = 8-amino-3,6-dioxaoctanoic acids; flu = 5(6)-carboxy-fluorescein)

### Bacterium strains

Overnight cultures of reference strains were prepared (American Type Culture Collection, Manassas, VA) representing various bacterial species all of which were Gram-negative rods (bacilli).

### Preparation of smears.

Smears were prepared as described in Example 1.

### Fluorescence in situ hybridization (FISH).

Hybridization was performed as described in Example 1 except the probes used were either Eco23S277-Flu at 500 nM, Abau23S-03b-Flu at 200 nM, or Abau16S-04c-Flu at 200 nM. Microscopic examination was conducted using a fluorescence microscope equipped with a FITC/Texas Red dual band filter set. Green fluorescent rods (bacilli) were identified as positives. Results are recorded in Table 4.

**Table 4**

| **Organism (ATCC#)** | **Eco23S277** | **Abau16S-04c** | **Abau23S-03b** |
|---|---|---|---|
| *Escherichia coli* (35218) | Positive/Green | Negative | Negative |
| *Escherichia coli (0157:H7)* (43888) | Positive/Green | Not done | not done |
| *Acinetobacter calcoaceticus* (14987) | Negative | Positive/Green | Positive/Green |
| *Acinetobacter baumannii* (19606) | Not done | Positive/Green | Positive/Green |
| *Pseudomonas aeruginosa* (10145) | Negative | Negative | Negative |
| *Klebsiella pneumoniae (*10031) | Negative | Negative | Negative |
| *Klebsiella* oxytoca (43086) | Negative | Negative | Negative |

With reference to Table 4, positive results were recorded for the Eco23S277-Flu probe tested against *Escherichia coli* isolates, and for both the Abau23S-03b-Flu and Abau23S-03b-Flu probes tested against *Acinetobacter* species, all other results were negative indicating that the probes/probe sets are specific.

## Claims

1. A PNA probe comprising a nucleobase sequence for the analysis of *Acinetobacter* species, wherein said PNA probe is SEQ ID NO: 8 or 9 and complements thereof.

2. The PNA probe of claim 1, wherein the probe is labeled with at least one detectable moiety.

3. The PNA probe of claim 2, wherein the detectable moiety or moieties are selected from the group consisting of: a conjugate, a branched detection system, a chromophore, a fluorophore, a spin label, a radioisotope, an enzyme, a hapten, an acridinium ester and a luminescent compound.

4. The PNA probe of claim 2, wherein the probe is self-reporting.

5. The PNA probe of claim 4, wherein the probe is a PNA Linear Beacon.

6. The PNA probe of claim 1, wherein the probe is unlabeled.

7. The PNA probe of claim 1, wherein the probe is bound to a support.

8. The PNA probe of claim 7, wherein the probe further comprises a spacer or a linker.

9. The PNA probe of claim 1, wherein *in situ* hybridization is used for analysis of *Acinetobacter* species optionally present in the sample.

10. A method for the detection, identification or quantitation of *Acinetobacter* species in a sample, said method comprising:
contacting at least one PNA probe of claim 1 to the sample;
hybridizing the PNA probe to a target sequence of *Acinetobacter* species in the sample; and
detecting the hybridization as being indicative of presence, identity and/or amount of *Acinetobacter* species in the sample.

11. A method according to claim 10, wherein the analysis takes place *in situ.*

12. A method according to claim 11, wherein the analysis takes place by fluorescence *in situ* hybridization.

13. A method according to claim 12, wherein the method does not involve use of cross-linking reagents or enzymes prior to hybridization.

14. The method of claim 12, wherein the method is used to detect a nucleic acid comprising a target sequence wherein said nucleic acid has been synthesized or amplified in a reaction.

15. The method of claim 14, wherein preferred nucleic acid synthesis or nucleic acid amplification reactions are selected from the group consisting of: Polymerase Chain Reaction (PCR), Ligase Chain Reaction (LCR), Strand Displacement Amplification (SDA), Transcription-Mediated Amplification (TMA), Rolling Circle Amplification (RCA) and Q beta replicase.

16. The method of claim 12, wherein the method further comprises adding at least one blocking probe to reduce or eliminate any hybridization of the PNA probe to non-target sequence.

17. The method of claim 16, wherein the target sequence is immobilized to a surface.

18. The method of claim 12, wherein said PNA probe is immobilized to a surface.

19. The method of claim 18, wherein said PNA probe is one component of an array.

20. The method of claim 12, wherein the sample is a biological sample.

21. The method of claim 20, wherein the biological sample is blood, urine, secretion, sweat, sputum, stool, mucous, or cultures thereof.

22. A kit for detection, identification or quantitation of one or more *Acinetobacter* species comprising one or more PNA probes set forth in claim 1 and instructions for use.

23. The kit of claim 22, wherein the kit is used in an *in situ* hybridization assay.

24. The kit of claim 22, wherein the kit is used for a real-time PCR assay.

25. The kit of claim 22, wherein the kit is used to examine clinical samples such as clinical specimens or cultures thereof.

## Patentansprüche

1. PNA-Sonde, die eine Nucleobasensequenz umfasst, für die Analyse von *Acinetobacter*-Spezies, wobei die PNA-Sonde SEQ ID NO: 8 oder 9 oder Komplemente davon ist.

2. PNA-Sonde nach Anspruch 1, wobei die Sonde mit wenigstens einer detektierbaren Gruppierung markiert ist.

3. PNA-Sonde nach Anspruch 1, wobei die detektierbare Gruppierung oder die detektierbaren Gruppierungen ausgewählt ist/sind aus der Gruppe, bestehend aus: einem Konjugat, einem verzweigten Detektionssystem, einem Chromophor, einem Fluorophor, einer Spin-Markierung, einem Radioisotop, einem Enzym, einem Hapten, einem Acridiniumester und einer lumineszierenden Verbindung.

4. PNA-Sonde nach Anspruch 2, wobei die Sonde "self-reporting" ist.

5. PNA-Sonde nach Anspruch 4, wobei die Sonde ein PNA-Linear-Beacon ist.

6. PNA-Sonde nach Anspruch 1, wobei die Sonde unmarkiert ist.

7. PNA-Sonde nach Anspruch 1, wobei die Sonde an einen Träger gebunden ist.

8. PNA-Sonde nach Anspruch 7, wobei die Sonde außerdem einen Spacer oder einen Linker umfasst.

9. PNA-Sonde nach Anspruch 1, wobei *in-situ-*Hybridisierung für die Analyse von *Acinetobacter-*Spezies, die gegebenenfalls in der Probe vorliegt, verwendet wird.

10. Verfahren für die Detektion, Identifizierung oder Quantifizierung von *Acinetobacter-*Spezies in einer Probe, wobei das Verfahren umfasst:
In-Kontaktbringen wenigstens einer PNA-Sonde nach Anspruch 1 mit der Probe;
Hybridisieren der PNA-Sonde an eine Zielsequenz von *Acinetobacter-*Spezies in der Probe und
Detektieren der Hybridisierung als Hinweis für das Vorliegen, die Identität und/oder die Menge von *Acinetobacter-Spezies* in der Probe.

11. Verfahren nach Anspruch 10, wobei die Analyse *in situ* stattfindet.

12. Verfahren nach Anspruch 11, wobei die Analyse durch Fluoreszenz-in-situ-Hybridisierung erfolgt.

13. Verfahren nach Anspruch 12, wobei das Verfahren keine Verwendung von Vernetzungsreagenzien oder Enzymen vor Hybridisierung involviert.

14. Verfahren nach Anspruch 12, wobei das Verfahren verwendet wird, um eine Nucleinsäure, die eine Zielsequenz umfasst, zu detektieren, wobei die Nucleinsäure in einer Reaktion synthetisiert oder amplifiziert wurde.

15. Verfahren nach Anspruch 14, wobei bevorzugte Nucleinsäure-Synthese- oder Nucleinsäure-Amplifikations-Reaktionen ausgewählt sind aus der Gruppe, bestehend aus: Polymerase-Kettenreaktion (PCR), Ligase-Kettenreaktion (LCR), Strang-Verdrängungs-Amplifikation (SDA), Transkriptions-vermittelte Amplifikation (TMA), Rolling-Circle-Amplifikation (RCA) und Q-beta-Replikase.

16. Verfahren nach Anspruch 12, wobei das Verfahren außerdem Anfügen wenigestens einer Blockierungssonde umfasst, um eine Hybridisierung der PNA-Sonde an nicht-ZielSequenz zu verringern oder zu eliminieren.

17. Verfahren nach Anspruch 16, wobei die Zielsequenz an einer Oberfläche immobilisiert wird.

18. Verfahren nach Anspruch 12, wobei die PNA-Sonde an einer Oberfläche immobilisiert wird.

19. Verfahren nach Anspruch 18, wobei die PNA-Sonde eine Komponente eines Arrays ist.

20. Verfahren nach Anspruch 12, wobei die Probe eine biologische Probe ist.

21. Verfahren nach Anspruch 20, wobei die biologische Probe Blut, Urin, Sekretion, Schweiß, Sputum, Stuhl, Schleim oder Kulturen davon ist.

22. Kit zur Detektion, Identifizierung oder Quantifizierung einer oder mehrerer *Acinetobacter-*Spezies, der eine oder mehrere PNA-Sonde(n), wie in Anspruch 1 angegeben, und Anweisungen zur Verwendung umfasst.

23. Kit nach Anspruch 22, wobei der Kit in einem *in-situ*-Hybridisierungs-Assay verwendet wird.

24. Kit nach Anspruch 22, wobei der Kit für einen Echtzeit-PCR-Assay verwendet wird.

25. Kit nach Anspruch 22, wobei der Kit verwendet wird, um klinische Proben, z.B. klinische Untersuchungsproben oder Kulturen davon zu untersuchen.

## Revendications

1. Sonde APN comprenant une séquence de bases nucléiques pour l'analyse d'espèces d'*Acinetobacter,* dans laquelle ladite sonde APN est SEQ ID NO : 8 ou 9 et leurs compléments.

2. Sonde APN selon la revendication 1, dans laquelle la sonde est marquée avec au moins un fragment détectable.

3. Sonde APN selon la revendication 2, dans laquelle le ou les fragments détectables sont choisis dans le groupe constitué : d'un conjugué, d'un système ramifié de détection, d'un chromophore, d'un fluorophore, d'un marqueur de spin, d'un radio-isotope, d'une enzyme, d'un haptène, d'un ester d'acridinium et d'un composé luminescent.

4. Sonde APN selon la revendication 2, dans laquelle la sonde est un marqueur en elle-même.

5. Sonde APN selon la revendication 4, dans laquelle la sonde est une balise linéaire à APN.

6. Sonde APN selon la revendication 1, dans laquelle la sonde n'est pas marquée.

7. Sonde APN selon la revendication 1, dans laquelle la sonde est liée à un support.

8. Sonde APN selon la revendication 7, dans laquelle la sonde comprend en outre un espaceur ou un lieur.

9. Sonde APN selon la revendication 1, dans laquelle une hybridation *in situ* est utilisée pour l'analyse de l'espèce d'*Acinetobacter* éventuellement présente dans l'échantillon.

10. Procédé de détection, d'identification ou de quantification d'une espèce d'*Acinetobacter* dans un échantillon, ledit procédé comprenant :
le contact d'au moins une sonde APN de la revendication 1 avec l'échantillon ;
l'hybridation de la sonde APN à une séquence cible de l'espèce d'*Acinetobacter* se trouvant dans l'échantillon ; et
la détection de l'hybridation en tant qu'indication de la présence, de l'identité et/ou de la quantité de l'espèce d'*Acinetobacter* se trouvant dans l'échantillon.

11. Procédé selon la revendication 10, dans lequel l'analyse est réalisée *in situ.*

12. Procédé selon la revendication 11, dans lequel l'analyse est réalisée par une hybridation *in situ* de fluorescence.

13. Procédé selon la revendication 12, dans lequel le procédé n'implique pas l'utilisation de réactif de réticulation ou d'enzyme avant l'hybridation.

14. Procédé selon la revendication 12, dans lequel le procédé est utilisé pour détecter un acide nucléique comprenant une séquence cible, dans lequel ledit acide nucléique a été synthétisé ou amplifié dans une
réaction.

15. Procédé selon la revendication 14, dans lequel les réactions préférées de synthèse ou d'amplification d'acides nucléiques sont choisies dans le groupe constitué : de l'amplification en chaîne par polymérase (PCR), de l'amplification en chaîne par ligase (LCR), de l'amplification par déplacement de brin (SDA), de l'amplification médiée par la transcription (TMA), de l'amplification par cercle roulant (RCA) ou de l'amplification au moyen de la réplicase Q-bêta.

16. Procédé selon la revendication 12, dans lequel le procédé comprend en outre l'ajout d'au moins une sonde de blocage pour réduire ou éliminer toute hybridation de la sonde APN à une séquence non ciblée.

17. Procédé selon la revendication 16, dans lequel la séquence cible est immobilisée sur une surface.

18. Procédé selon la revendication 12, dans lequel ladite sonde APN est immobilisée sur une surface.

19. Procédé selon la revendication 18, dans lequel ladite sonde APN est un composant d'un réseau.

20. Procédé selon la revendication 12, dans lequel l'échantillon est un échantillon biologique.

21. Procédé selon la revendication 20, dans lequel l'échantillon biologique est un échantillon de sang, d'urine, de sécrétion, de sueur, de crachat, de selle ou de mucus ou leurs cultures.

22. Kit de détection, d'identification ou de quantification d'une ou de plusieurs espèces d'*Acinetobacter*, comprenant une ou plusieurs sondes APN selon la revendication 1 et des instructions pour l'utiliser.

23. Kit selon la revendication 22, dans lequel le kit est utilisé dans un essai d'hybridation *in situ.*

24. Kit selon la revendication 22, dans lequel le kit est utilisé pour un essai par PCR en temps réel.

25. Kit selon la revendication 22, dans lequel le kit est utilisé pour examiner des échantillons cliniques, tels que des spécimens cliniques ou leurs cultures.
